# EUROPEAN PATENT APPLICATION

(11) **EP 1 798 574 A1**
(43) Date of publication of application: **20.06.2007**
(21) Application number: 06023485.3
(22) Date of filing: 10.11.2006
(51) Int. Cl.: G01T 1/24, G01T 1/29

(54) **Nuclear medicine imaging system and method**

(30) Priority: 16.11.2005 JP 2005331008
(71) Applicant: HITACHI, LTD., Chiyoda-ku Tokyo 100-8280 (JP)
(72) Inventor: Seino, Tomoyuki Hitachi, Ltd., 1-chome Chiyoda-ku Tokyo 100-8220 (JP); Yokoi, Kazuma Hitachi, Ltd., 1-chome Chiyoda-ku Tokyo 100-8220 (JP); Takahashi, Isao Hitachi, Ltd., 1-chome Chiyoda-ku Tokyo 100-8220 (JP); Ueno, Yuichiro Hitachi, Ltd., 1-chome Chiyoda-ku Tokyo 100-8220 (JP)
(74) Representative: Strehl Schübel-Hopf & Partner

(57) **Abstract**

A nuclear medicine imaging system is configured so that a semiconductor element (S) and a metallic conductive member (22, 23) are bonded to one another with an electrically conductive adhesive (21A) composed of electric conductive particles and a resin binder, and a charge which is generated when radiation is incident on the semiconductor element (S) is taken as a signal by a detection circuit (30) from the conductive member (22, 23) through the electrically conductive adhesive (21A), and a current source unit (40) for forcing a larger current than that due to a charge generated by incoming radiation to flow at least through the electrically conductive adhesive (21A) and a protection circuit (31) for protecting the detection circuit (30) from the larger current passed by the current source unit (40) are provided.

## Description

### BACKGROUND OF THE INVENTION

The present invention relates to a nuclear medicine imaging system having a semiconductor radiation detector including a semiconductor element and a method thereof.

Recently, a nuclear medicine imaging system such as a gamma camera, a single photon emission computed tomographic equipment (SPECT equipment) and a positron-emission tomographic equipment (PET equipment) has been known as a system in which a radiation counting technology is applied for the medical field. For a radiation detector used in these systems, it is mainly of combination of a scintillator and photomultiplier tube. While, for example in JP-A-2004-317140, usage of a semiconductor other than the scintillator as a radiation detector is disclosed. In such a semiconductor radiation detector, an energy of radiation can be obtained correctly by measuring a quantity of electric charge, because such a radiation detector is configured to create an electron and hole due to the photoelectric effect produced by gamma radiation incident on a semiconductor element crystal and transfer the electron and hole by an electric field generated through an externally applied voltage so that the charge can be monitored externally, and this quantity of electric charge is proportional to the energy of the radiation.

Now, a method in which cadmium telluride (CdTe) is used as a semiconductor for detecting radiation, especially gamma radiation is known. Also, silicon or germanium may be used, but these are frequently used for detection of an X-ray. As for radioactive material used in the nuclear medicine diagnosis, for example technetium or fluorine 18 is known, and these have a high penetrating power because of energy of 141 kev or 511 kev. Therefore, it is more advantageous to use a material having the larger atomic number for the radiation detector, because it absorbs more gamma radiation to create a signal. From this point, CdTe has an advantage that it is excellent in absorption of gamma radiation due to the large average atomic number.

However, there are difficulties in CdTe that its crystal is brittle and it is difficult to deal with, and it may be damaged or have a defect caused inside the crystal by a slight shock or vibration. Then, when an electrically conductive member to be an electrode for collecting charges is connected to the crystal of CdTe, an electrically conductive adhesive is used for a connecting material.

However, after semiconductor radiation detectors are actually manufactured by laminating alternately what is formed from the conductive member and CdTe bonded to one another using the electrically conductive adhesive and used for several months for the purpose of experiment, then an increase in noise in a detection signal and a decrease in resolution of energy take place in a small number of the semiconductor radiation detectors.

It is guessed that these phenomena are due to an unstable electric contact caused by lowering in contractile force and oxidization of electrically conductive particles in the electrically conductive adhesive for bonding the conductive member to CdTe because of the long-period usage.

While, charges created in the semiconductor radiation detector by incoming radiation are very small to the extent of a few femtocoulombs to several dozen femtocoulombs, and a value of electric current may not reach the degree of a microampere. Therefore, a distortion in a signal may occur when such a faint signal passes through a portion of the unstable electric contact, and this may exhibit deterioration in the semiconductor radiation detector to lead to a decrease in resolution of energy.

### SUMMARY OF THE INVENTION

Therefore, an object of the present invention is to provide a nuclear medicine imaging system excellent in resolution of energy and a method thereof.

The present invention, to achieve the object described above, provides a configuration including a current source unit that forces a larger current than that created by charges generated through incoming radiation to flow at least through an electrically conductive adhesive. According to this configuration, recombination of electrically conductive particles of the electrically conductive adhesive and destruction of an oxidized film formed in the electrically conductive adhesive can be performed by the large current passed by the current source unit, to provide stabilization of electric contact properties of the electrically conductive adhesive. Also, because of including a protection circuit, a detection circuit can be protected from the current passed by the current source unit to improve reliability along with stabilization of the electric contact properties of the electrically conductive adhesive. Further, supplying electricity by the current source unit before applying a voltage for measurement may effectively suppress occurrence of failure caused by the unstable electric contact to maintain performance of detection of radiation over a long period of time.

Further, in the case of a configuration in which a semiconductor element having diode properties and a metallic conductive member are bonded to one another by an electrically conductive adhesive, recombination of electrically conductive particles of the electrically conductive adhesive and destruction of an oxidized film formed in the electrically conductive adhesive can be carried out by forcing a larger forward current through a current source unit than that created by charges due to incoming radiation to flow, to provide stabilization of electric contact properties of the electrically conductive adhesive.

Moreover, it is easy at a low cost to provide a protection circuit, because it is configured from any one of a zener diode, selector switch and varistor intervening between a signal output side of the semiconductor element and the ground side, or any combination thereof. Especially in a nuclear medicine imaging system requiring many semiconductor elements, minimum required addition to a configuration may allow performance of detection of radiation to be maintained in a better condition.

The present invention provides a nuclear medicine imaging system excellent in resolution of energy and a method thereof.

Other objects, features and advantages of the invention will become apparent from the following description of the embodiments of the invention taken in conjunction with the accompanying drawings.

### BRIEF DESCRIPTION OF THE DRAWINGS

Fig. 1 is a perspective view in schematic form for illustrating a configuration of a PET imaging device suitable for a nuclear medicine imaging system of an embodiment 1 according to the present invention;
Fig. 2 is a perspective view in schematic form for illustrating the PET imaging device;
Fig. 3A is an elevation view of a unit board used in the PET imaging device shown in Fig. 2;
Fig. 3B is a side view of the unit board;
Fig. 4A is a perspective view in schematic form for illustrating a semiconductor radiation detector element;
Fig. 4B is a perspective view in schematic form for illustrating a detector using the semiconductor radiation detector element;
Fig. 4C is a perspective view in schematic form for illustrating a situation in which the detector is disposed on the board;
Fig. 5 is an enlarged view of the detector viewed from the incoming direction of gamma radiation;
Fig. 6 is a schematic circuit diagram for illustrating a distinguishing portion of the embodiment 1;
Fig. 7A is a schematic diagram for illustrating an example of arrangement for a light source;
Fig. 7B is a schematic diagram for illustrating an example of arrangement for the light source;
Fig. 8 shows a graph for illustrating relation between occurrence of failure in the detector and the number of days; and
Fig. 9 is a schematic circuit diagram for illustrating a distinguishing portion of an embodiment 2.

### DESCRIPTION OF THE INVENTION

Now, a PET equipment of a preferred embodiment for a nuclear medicine imaging system of the present invention will be explained in detail referencing properly to drawings.

### (Embodiment 1)

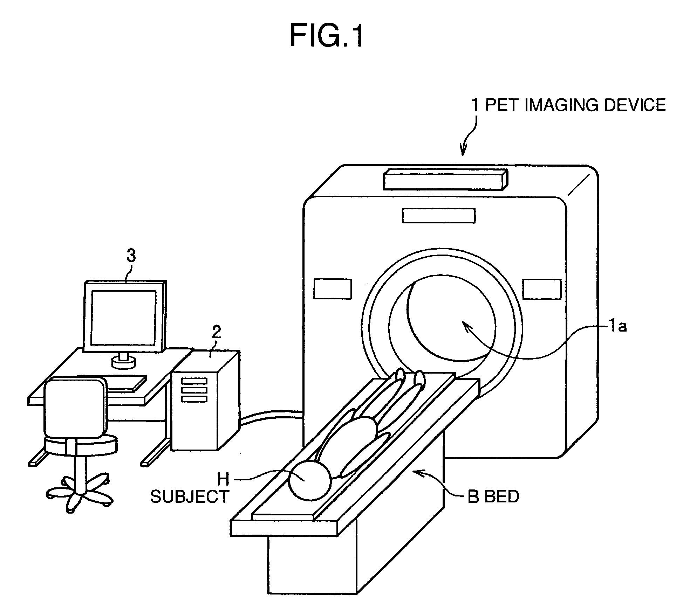

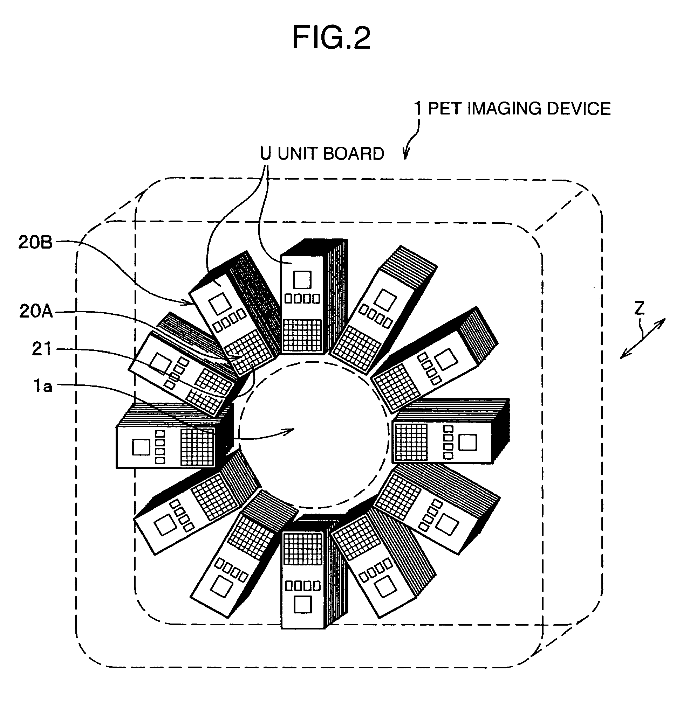

A PET equipment of this embodiment, as shown in Fig. 1, includes a PET imaging device 1 having a field of view 1a, a bed B for supporting a subject (examinee) H, a data processing device (for example, a computer) 2 and a display 3. The PET imaging device 1 has many unit boards U disposed in the circumferential direction as shown in Fig. 2, and in such PET imaging device 1, the subject H is, as shown in Fig. 1, placed on the movable bed B in its longitudinal direction and inserted into the cylindrical field of view 1a surrounded by the unit boards U.

### (PET imaging device)

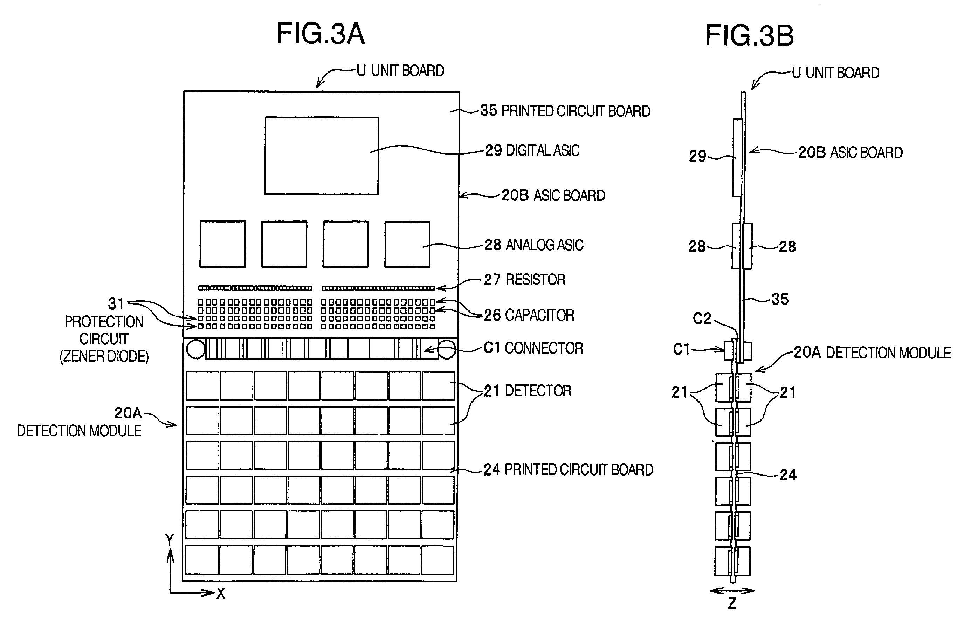

The PET imaging device 1 has many unit boards U disposed in the circumferential direction and surrounding the field of view 1a into which the bed B is inserted as described above. A plurality of unit boards U, as shown in Fig. 2, are also disposed in the longitudinal direction of the bed B (the direction denoted by arrow Z in Fig. 2). Each of the unit boards U includes, as shown in Figs. 3A and 3B, a radiation detection module 20A (hereinafter, referred to as "detection module") and an integrated circuit board 20B (hereinafter, referred to as "ASIC board"). The detection module 20A includes a plurality of semiconductor radiation detectors 21 (hereinafter, simply called "detector"). Each of the detectors 21 detects gamma radiation emitted from the inside of the subject H (see Fig. 1). The detector 21 will be described in detail below.

The ASIC board 20B includes integrated circuits (an analog ASIC 28 and digital ASIC 29) for measuring a pulse height value of the detected gamma radiation and detection time, and will measure the value of pulse height of the detected gamma radiation and detection time. The integrated circuits include a plurality of signal processing devices for processing a radiation detection signal.

Next, the PET imaging device 1 will be explained in detail.

### (Semiconductor radiation detector)

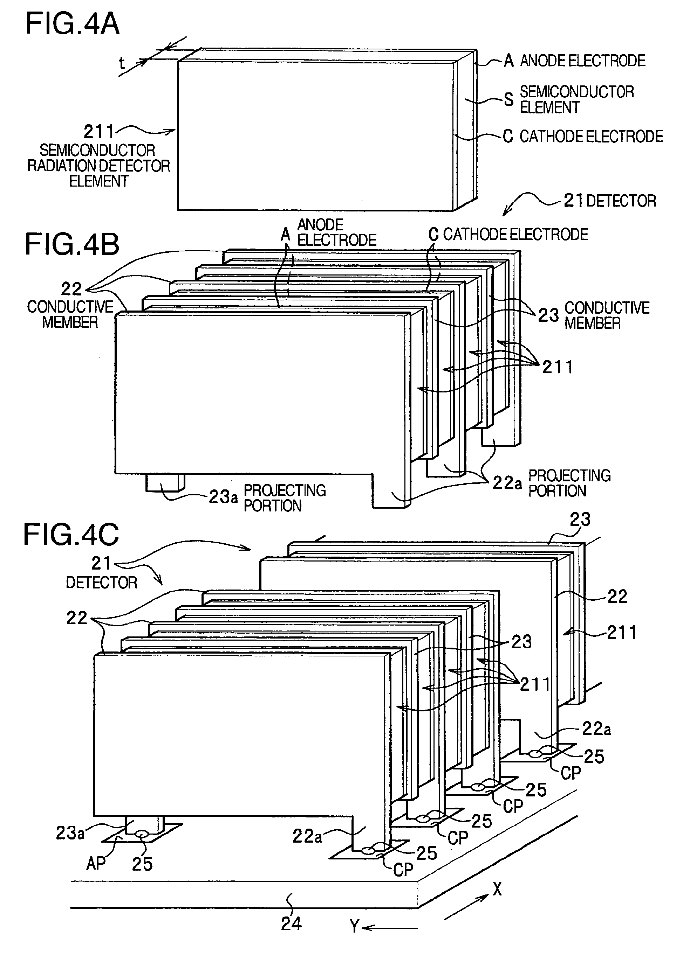

First, the detectors 21 applied to this embodiment will be explained. As shown in Figs. 4B and 4C, each of the detectors 21 includes four semiconductor radiation detector elements 211 (hereinafter, each of them is referred to as "detector element") and electrically conductive members 22, 23 disposed between the detector elements 211 and on both sides of the detector elements 211. The detector element 211 includes, as shown in Fig. 4A, a semiconductor element S composed of a plate-like semiconductor material and has thin film-like electrodes formed throughout on both sides thereof by evaporation deposition method and the like. An electrode formed on one of the sides is an anode electrode A (hereinafter, referred to as "anode"), and an electrode formed on the other side is a cathode electrode C (hereinafter, referred to as "cathode"). In each of the detectors 21, an even number (four in this embodiment) of the detector elements 211 of which the anodes A and cathodes C are vertically placed in the direction perpendicular to the mounting surface of a printed circuit board 24 (see Fig. 3A) and in a manner that the anodes A face one another and the cathodes C face one another are arranged in parallel in the directions perpendicular to radiation traveling direction (Y-direction and X-direction in Fig. 4C), and the electrodes of the same kind are electrically connected to one another (the anodes A are connected to one another and the cathodes C are connected to one another) through the electrically conductive members 22, 23.

The semiconductor element S is a region for creating charges by reacting with radiation and formed from any one of single crystals including CdTe, CdZnTe, GaAs and the like. Further, the cathode C and the anode A are formed from any one of materials including Pt, Au, In and the like. In this embodiment, in the detector element 211, a diode is configured, for example from the semiconductor element S using CdTe, the cathode C formed mainly from Pt and the anode A formed mainly from In.

Now, relation between time and a curve of the pulse height value will be explained for a thick and thin thickness t of the semiconductor element S (see Fig. 4A). When the same reverse bias voltage is applied to the junction between the cathode C and anode A, in the case of the thinner thickness t of the semiconductor element S, a rising rate (at rising edge) of the pulse height value is larger, and accuracy of the pulse height value (resolution of energy) is higher. A larger rising rate of the pulse height value, for example improves accuracy of coincident measurement (resolution of coincident counting) in the PET imaging device 1. The larger the rising rate of the pulse height value in the semiconductor element S having the thinner thickness t is, the higher the resolution of energy becomes (collection efficiency of charge becomes higher), and it is because the time required for an electron to reach the anode A and a hole to reach the cathode C are reduced, that is, collection time of charge becomes shorter. Further, it is because a hole which may disappear during traveling is allowed to reach the cathode C without disappearing due to the thinner thickness t. For reference's sake, the thickness t may be also expressed by a distance between the electrodes of the cathode C and anode A. Moreover, the anode A is an electrode for taking a radiation detection signal, and the cathode C is an electrode for applying the reverse bias voltage.

Further, the thickness t (the distance between the electrodes) of the semiconductor element S is preferably in the range of 0.2 to 2.0 mm. It is because if the thickness t is beyond 2.0 mm, the rising rate of the pulse height value will become smaller, and the maximum of it also becomes lower. Assuming that the thickness t is made thicker, the traveling speed of an electron and hole may be increased by raising the reverse bias voltage to increase the strength of an electric field in the depth direction t of the detection element 211, and accordingly the required time for an electron and hole to reach the respective electrodes can be decreased. However, the increase in the reverse bias voltage applied may be not preferred, because it may incur a down side, for example it may pose upsizing of a DC high voltage supply and lead to dielectric breakdown inside the printed circuit board 24 and the like. While, if the thickness t is below 0.2 mm, the thickness (volume) of the electrodes (anode A and cathode C) will relatively increase. Under such circumstances, proportion of the essential semiconductor element S which reacts with radiation becomes smaller. That is, if the thickness t of the semiconductor element S is made thinner, the thickness of the electrodes (the anode A and cathode C) which does not react with gamma radiation, that is, does not detect gamma radiation becomes relatively larger, while the proportion of the semiconductor element S which reacts with gamma radiation becomes smaller, and as the result, sensitivity of detecting gamma radiation goes lower. Also, when the thickness t is thin, capacitance per detector element 211 becomes larger. This capacitance corresponds to an input capacitive component viewed from a signal processing circuit (ASIC) in a latter stage, and therefore the larger the input capacity is, the more a noise is likely to arise in the signal processing circuit and resolution of energy or coincident counting tends to deteriorate. Further, in order to achieve detection sensitivity per detector 21 to some extent, a volume of the detector 21 is obtained effectively by disposing the detector elements 211 in parallel, therefore the thinner the thickness t is, the larger the number of elements disposed in parallel has to be. As the result, a synergistic increase in capacitance per detector 21 may incur performance deterioration in the PET imaging device 1 (for example, deterioration in contrast of PET imaging due to deterioration in resolution of energy, and an increase in the inspection time or deterioration in image quality due to deterioration in resolution of coincident counting). Therefore, the thickness t described above is preferred.

The electrically conductive member 22, 23 is a plate-like member formed from at least one of the group composed of iron-nickel alloy, iron-nickel-cobalt alloy, chromium and tantalum. Further, 42 alloy (Fe 58% and Ni 42%) may be used for iron-nickel alloy, and Kovar (Fe 54%, Ni 29% and Co 17%) may be used for iron-nickel-cobalt alloy.

In this embodiment, the conductive member 22, 23 is sized sufficiently to cover an area of the respective electrodes of the detector element 211, that is, formed larger than the area of the respective electrodes. However, the size of the conductive member 22, 23 may be the same size as the detector element 211. Also, the thickness of the conductive member 22, 23 is in the range of about 10 µm to about 100 µm and preferably chiefly about 50 µm. Such conductive member 22, 23 includes, as shown in Fig. 4C, a projecting portion 22a, 23a extending below the detector element 211 (to the side of the printed circuit board 24) in an attached situation to the detector element 211. The projecting portion 22a, 23a acts as a fixing portion to attach the detector 21 to the printed circuit board 24, and the projection portion 22a is connected to a connecting member CP for the cathode C disposed on the printed circuit board 24, and the projection portion 23a is connected to a connecting member AP for the anode A disposed on the printed circuit board 24. Also, the detector 21 is attached to the printed circuit board 24 out of touch, i.e. attached with the detector element 211 having a predetermined clearance from the printed circuit board 24 by the projecting portion 22a, 23a. This can suitably prevent lowering in insulation performance caused by dust and the like pinched between the detector 21 and printed circuit board 24 upon attachment. Also, enhanced air permeability through the clearance may allow the detector 21 to be cooled to stabilize characteristics of the detector 21. Moreover, the bottom surface of the detector 21 may be coated with insulating material (not shown), thereby further preventing occurrence of unexpected dielectric breakdown.

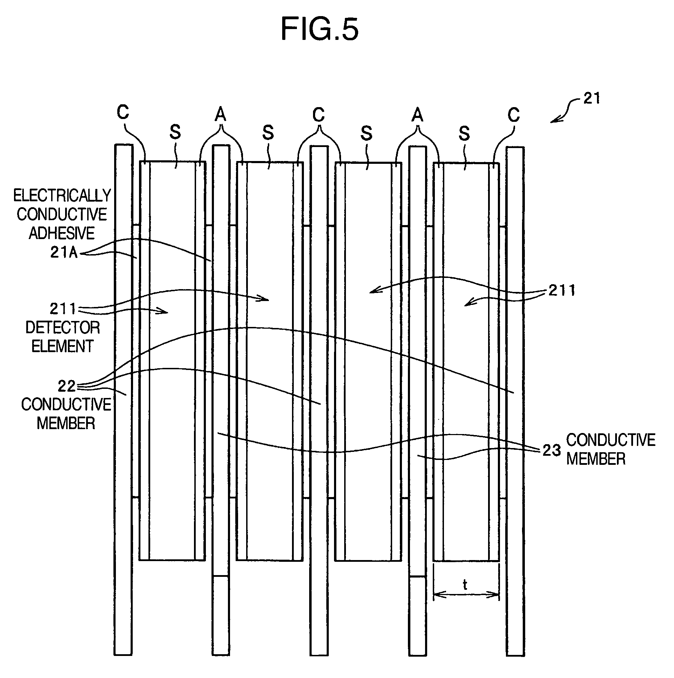

Such conductive member 22, 23 is, as shown in Fig. 5, bonded and attached to the detector element 211 with an electrically conductive adhesive 21A. As for the electrically conductive adhesive 21A, for example an adhesive formed from an insulating resin binder composed of organic polymer material and into which electrically conductive particles such as metal powder (silver) are dispersed may be used. Usually, when the detector element 211 and conductive member 22, 23 are bonded to one another with the electrically conductive adhesive 21A, heat-treatment at a high temperature of about 120 to about 150°C is carried out to cure the electrically conductive adhesive 21A.

In this embodiment, each of the semiconductor elements S disposed in parallel has the thickness t described above (0.2 to 2.0 mm), and the thickness of the cathode C and anode A is set to some microns about the most. For a plurality of the detector elements 211, the cathodes C are commonly connected to one another and the anodes A are commonly connected to one another, therefore in the detector 21, it is unknown what semiconductor element S in the detector element 211 reacts with gamma radiation. The detector 21 is configured as described above, because such configuration can allow the thickness t of the semiconductor element S to be thinner to improve collection efficiency of charge, increase the rising rate of the pulse height value to improve resolution of energy and further decrease an amount of gamma radiation passing through without reaction due to the parallel arrangement of the semiconductor elements S to increase occurrence of reaction between the semiconductor element S and gamma radiation (to increase the count number of gamma radiation). The increase in the count number of gamma radiation improves sensitivity of the detector 21.

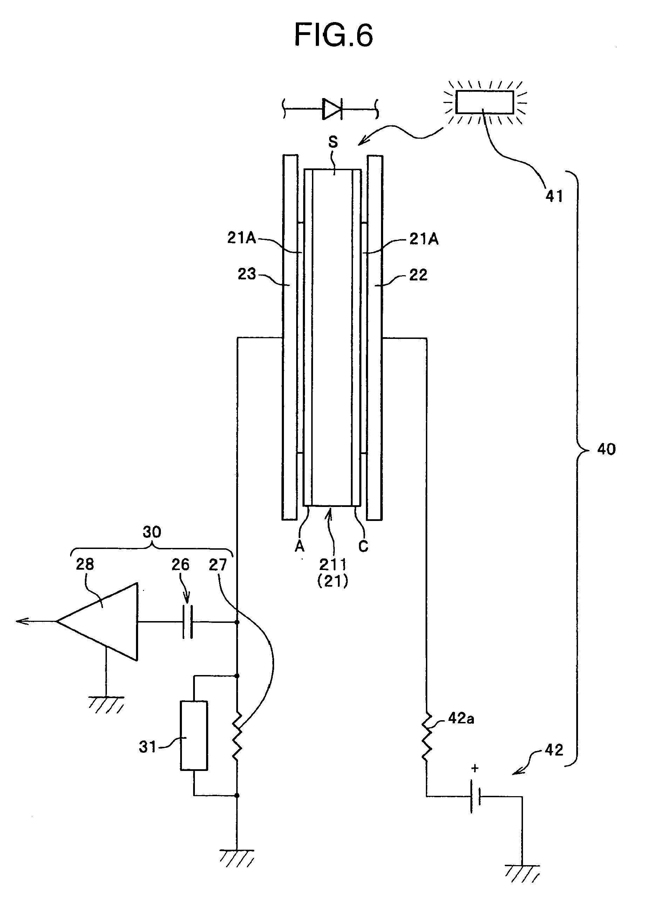

Now, a principle for detecting gamma radiation by using the detector 21 will be explained briefly. When incoming gamma radiation (not shown) reacts with the semiconductor element S, a pair of a hole and electron is created by the quantity proportional to energy of gamma radiation. Also, a reverse bias voltage (for example, a reversely applied voltage formed from a potential of -500 V at the cathode C and a potential at the anode A which is close to the ground potential, i.e. higher by 500 V than that at the cathode C) supplied by a DC high voltage supply 42 described below (see Fig. 6) for collecting charges is applied between the electrodes of the cathode C and anode A of the detector element 211 composing the detector 21. Therefore, a hole corresponding to a positive charge is drawn to travel toward the cathode C, and an electron corresponding to a negative charge is drawn to travel toward the anode A. Because mobility of an electron is relatively higher than that of a hole, comparing an electron to a hole, an electron travels to reach the anode A relatively in a short period of time. While, because mobility of a hole is relatively lower, a hole travels to reach the cathode C relatively in a long period of time. For reference's sake, an electron or hole may be trapped during traveling before arrival at the anode A or cathode C.

The conductive member 22 and conductive member 23 are non-sensitive areas which does not detect gamma radiation. Therefore, in the detector 21, the conductive member 22, 23 which is non-sensitive area is disposed between the detector elements 211. Also, the anode A and cathode C are non-sensitive areas.

As shown in Figs. 3A and 3B, such detectors 21 are disposed by 6 channels of the detector 21 in the Y-direction from the detection module 20A to the ASIC board 20B (the radial direction in the PET imaging device 1, see Fig. 2), by 8 channels in the X-direction perpendicular to the Y-direction (the circumferential direction in the PET imaging device 1, see Fig. 2) and further by 2 channels (on both sides of the printed circuit board 24) in the Z-direction, i.e. thickness direction of the printed circuit board 24 (the depth direction in the PET imaging device 1, see Fig. 2) on the printed circuit board 24 of the detection module 20A. Then, there is a total of 96 channels of the detector 21 disposed summing up a total of 48 channels on one side of the printed circuit board 24 and a total of 48 channels on the other side.

In this embodiment, in order to maintain detection performance of gamma radiation of each of such detectors 21, as shown in Fig. 6, a current source unit 40 for securing electric contact properties of the electrically conductive adhesive 21A and a protection circuit 31 for protecting a detection circuit 30 described below from current passed due to an effect of the current source unit 40 are provided. However, in Fig. 6, taking one detector element 211 out of the detectors 21 as an example, an explanation will be provided.

As described above, incoming gamma radiation on the detector element 211 of the detector 21 induces current due to charges to flow, and the current source unit 40 serves to force a larger current than that flowing at this time to flow at least through the electrically conductive adhesive 21A. In order to achieve this, the current source unit 40 includes a light source 41 for irradiating the detector element 211 of the detector 21 with a light beam, and a high voltage supply (circuit) 42 for applying a reverse bias voltage to the detector element 211. That is, the current source unit 40 serves to irradiate the detector 21 with a strong light beam by using the light source 41 to force a larger current than that induced by incoming gamma radiation to flow through the detector element 211, and by passing a high current through prior to an actual measurement, therefore it is achieved to improve a condition of electric contact mainly in the electrically conductive adhesive 21A.

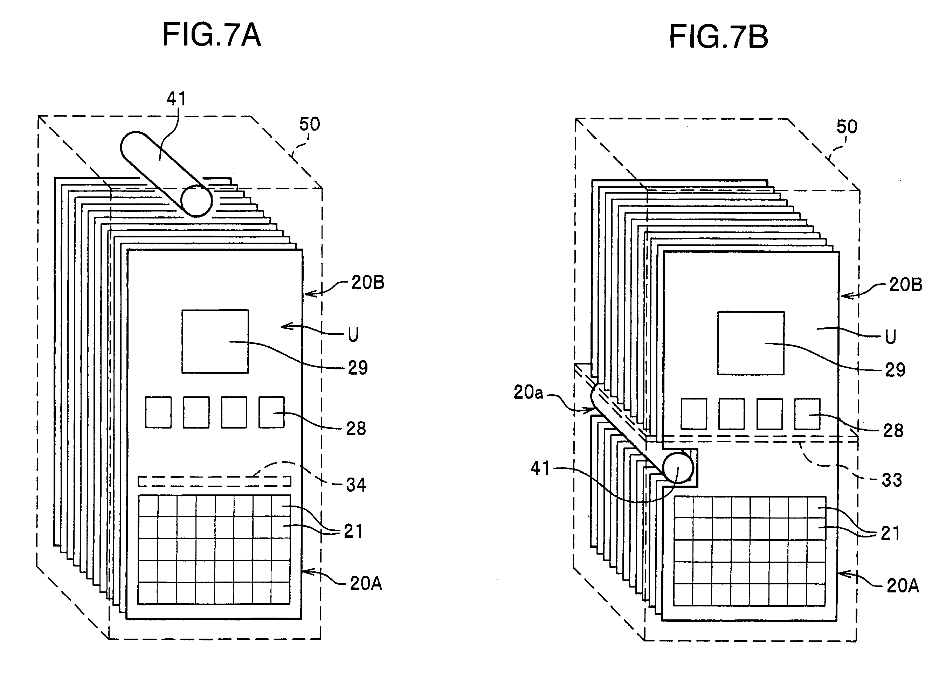

The light source 41 may be such that it can emit a light beam sufficient to cause a larger current than that induced by incoming gamma radiation on the detector element 211 to flow through the electrically conductive adhesive 21A, and in this example, a fluorescent bulb is used. However, the light source 41 is not to be limited to the fluorescent bulb, and various light sources which emit a light beam with a wavelength not more than 800 nm may be employed for the light source 41. As shown in Fig. 7A, in the case where a plurality of unit boards U are contained in a storage member 50, the light source 41 may be disposed in an open space on the upper side or the like in the storage member 50. Moreover, in the case where, for example a light-emitting diode or the like is used for the light source 41, the light source 41 may be disposed directly in a space 34 (shown by dashed lines) etc. on the unit board U. Also, the light source 41 (the fluorescent bulb and the like) may be disposed, as shown in Fig. 7B, in a space 20a formed by cutting in the side of the unit board U (a space close to the detection module 20A). Because of this arrangement, it can be allowed to well irradiate each of the detectors 21 in a lower area (an area in which the detection modules 20A are disposed) with a light beam, even if an upper area (an area in which the ASIC boards 20B are disposed) is separated from the lower area by a barrier portion 33 in the storage member 50 for thermal insulation and the like.

The high voltage supply 42 is, as shown in Fig. 6, connected to the side of the cathode C of the detector element 211 through a resistor 42a and applies a reverse bias voltage to the detector element 211. In this embodiment, the high voltage supply 42 which is usually provided in the PET imaging device 1 (see Fig. 1) to drive the detectors 21 is used also as power supply for the current source unit 40. That is, an increase in cost can be suppressed, because it is not necessary to provide a special power supply to improve the condition of the electric contact in the electrically conductive adhesive 21A.

The side of the anode A of the detector element 211 of the detector 21 is connected to an analog ASIC 28 through a capacitor 26 and also to the side of the ground through a resistor 27. In this embodiment, a protection circuit 31 is connected to this resistor 27 in parallel. Due to this, the side of the anode A of the detector element 211 is also connected to the side of the ground through the protection circuit 31. The detection circuit 30 includes the analog ASIC 28, capacitor 26 and resistor 27 and detects current flowing through the detector 21 when radiation is incident.

The protection circuit 31 serves to avoid an excessive voltage being applied to the resistor 27 to prevent a voltage across the capacitor 26 from rising beyond a voltage rating. In this example, a zener diode is used for the protection circuit 31. However, the protection circuit 31 is not to be limited to the zener diode, but in place of or combination with this, a selector switch or varistor may be used. In the case of the selector switch, by forming a short circuit in the selector switch between the anode A which is a signal output side of the detector element 211 and the ground during an ON state of the high voltage supply 42, the same effect as the case of using the zener diode can be achieved.

### (Unit board)

The unit boards U are, as shown in Fig. 2, disposed on an annular supporting member (not shown) provided in the PET imaging device 1 in such a manner that a surface on which the detectors 21 are placed faces the depth direction (the Z-direction) of the PET imaging device 1. A plurality of the unit boards U disposed on the annular supporting member are provided circumferentially and surround the field of view 1a. Further, they are disposed so that the detection modules 20A is situated on the inside (on the side of the field of view 1a) and the ASIC boards 20B is situated on the outside. In this embodiment, a plurality of the unit boards U are also disposed in the depth direction of the PET imaging device 1.

The unit boards U will be explained in detail in relation to their configuration with reference to Figs. 3A and 3B. Each of the unit boards U includes the detection module 20A on which a plurality of the detectors 21 are disposed as described above and the ASIC board 20B. The ASIC board 20B includes the capacitor 26, resistor 27, analog ASIC 28 and digital ASIC 29. Further, the zener diode as the protection circuit 31 is disposed between the detection module 20A and the capacitor 26 on the ASIC board 20B. A detection signal of gamma radiation output from each of the detectors 21 is supplied from the side of the detection module 20A to the side of the ASIC board 20B through a connector C1.

The ASIC board 20B has, as shown in Figs. 3A and 3B, one digital ASIC 29 disposed on one of the surfaces and four analog ASICs 28 disposed on both of the surfaces. On both of the surfaces of the ASIC board 20B, the capacitors 26, resistors 27 and zener diodes (protection circuits 31) are disposed by the number corresponding to the number of the detector 21.

Also, a plurality of wiring lines (not shown) are provided in the ASIC board 20B to electrically connect these zener diodes (protection circuits 31), capacitors 26, resistors 27, analog ASICs 28 and digital ASIC 29. Each of the analog ASICs 28 means an ASIC (Application Specific Integrated Circuit) which is an IC for a special application and processes an analog signal (a detection signal of gamma radiation) output from the detector 21, and it is a kind of a LSI. The analog ASIC 28 has a signal processing circuit provided for each of the detectors 21. Each of these signal processing circuits receives a detection signal of gamma radiation (a detection signal of radiation) output from one corresponding detector 21 to derive the pulse height value of the gamma radiation.

A shorter length of a circuit and a shorter length of a wiring line for transmitting the detection signal of gamma radiation are preferred, because the signal may be less affected by a noise and less decayed during traveling. Further, in the case where coincident measuring process is carried out in the PET imaging device 1, the shorter length of the circuit or wiring line is preferred because of a smaller time delay (it is preferred, because accuracy of detecting time is not impaired). For this purpose, in this embodiment, the detector 21, zener diode (protection circuit 31), capacitor 26, analog ASIC 28 and digital ASIC 29 are disposed in this order on the unit board U radially outward from the central axis in the PET imaging device 1. This arrangement can allow a wiring line for transmitting a faint detection signal of gamma radiation output from the detector 21 to an amplifier in the analog ASIC 28 to be made shorter. Therefore, an effect of a noise on the detection signal of gamma radiation may be reduced, and attenuation of the detection signal of gamma radiation may be lowered.

Alternately, the zener diode (the protection circuit 31), capacitor 26, resistor 27, analog ASIC 28 disposed on the ASIC board 20B may be disposed on the detection module 20A other than the ASIC board 20B. In that case, the zener diode (the protection circuit 31), capacitor 26, resistor 27, analog ASIC 28 is situated on the side of the ASIC board 20B away from the detector 21. However, because the detection module 20A includes the detector 21 and analog ASIC 28, a distance between the detector 21 and analog ASIC 28 (length of a wiring line) may be further reduced. Therefore, the effect of a noise may be further lowered.

### (Operation of PET imaging device)

Operation of the PET imaging device 1 configured as described above at the time of measurement will be explained, mainly with reference to Fig. 6.

First, operation at a normal measurement will be explained, and then operation at startup of the PET imaging device 1 in relation to a distinguishing portion of this embodiment will be described.

First, the PET imaging device 1 is activated by manipulating an operation switch (not shown), then a reverse bias voltage is applied to the detector 21 through the resistor 42a by the high voltage supply 42. At this time, because the detector element 211 in the detector 21 has a diode characteristic, a leakage current is generated in the detector element 211 due to the reverse bias voltage applied, but the leakage current per detector element 211 is in the range of some nanoamperes to several dozen nanoamperes, and a total of the leakage current in the detector 21 is a very small current to the extent of a few hundred nanoamperes.

Under this circumstance, during measurement, when gamma radiation incident on the detector 21 causes the photoelectric effect, a carrier composed of an electron and hole is created. The carrier is moved by an electric field generated by the reverse bias voltage and taken as a charge signal on the side of the anode A. Specifically, current corresponding to a quantity of charge flows through the detector 21 and is stored in the capacitor 26. Subsequently, the charge stored is discharged to the side of the ground through the resistor 27 and a voltage on the side of the analog ASIC 28 of the detector 21 returns to zero. A change in voltage across the capacitor 26 due to storage of charge forms a high frequency signal of not more than a microsecond, and therefore the current stored in the capacitor 26 passes through the capacitor 26 to reach the analog ASIC 28. Accordingly, measurement of timing at which gamma radiation is incident and energy is carried out by amplifying this detection signal in the analog ASIC 28 and processing the signal in the digital ASIC 29 in a latter stage (see Fig. 3A).

The foregoing is the operation at the normal measurement.

As described above, the detection signal output from the detector element 211 is sent from the anode A to the conductive member 23 through the electrically conductive adhesive 21A. The detection signal is a high frequency signal of not less than 10 MHz, therefore, if a portion of defective electric contact in the electrically conductive adhesive 21A is present, a distortion is generated when the detection signal passes through that portion, which may appear as the same deterioration in characteristics as that caused by an increase in noise. Therefore, it is required to maintain a better condition of the electric contact in the electrically conductive adhesive 21A, and for that purpose, operation at startup explained below becomes necessary.

First, before starting the PET imaging device 1, the reverse bias voltage is applied to the detector 21 by activating the high voltage supply 42 through operation on a control panel (not shown) and the like, and a fluorescent bulb as the light source 41 (current source unit 40) is turned on for a predetermined period of time. In this example, the fluorescent bulb is turned on for about 10 sec with the voltage being applied. Further, these operations may be automatically performed according to a preset program (not shown), for example before a normal startup or every one week.

The lighted fluorescent bulb with the voltage being applied causes a larger current than that generated by incoming gamma radiation to flow in the detector element 211 of the detector 21. For that matter, the fluorescent bulb (the light source 41) is turned on in the storage member 50 shown in Fig. 7A, and then it is confirmed that a value of current obtained by averaging current which flows in each of a plurality of the detectors 21 contained in the storage member 50 is 10 microamperes. That is, current of 10 microamperes can be passed through the electrically conductive adhesive 21A.

Such a large amount of current flowing through the detector 21 may allow electrically conductive particles contained in the electrically conductive adhesive 21A to recombine with one another or an oxidized film formed in the electrically conductive adhesive 21A to be destroyed, to stabilize electric contact properties.

However, such a large amount of current flowing through the detector 21 may bring a voltage applied across the resistor 27 into a high voltage beyond 100 V, which may raise a voltage across the capacitor 26 up beyond a voltage rating thereof. Under this condition, in this embodiment, because the zener diode is used for the protection circuit 31, an effect of the zener diode may lower the voltage across the resistor 27, and as the result, the voltage across the capacitor 26 may be kept below the voltage rating. Therefore, it is not necessary to use en expensive capacitor of a high voltage rating. It can be intended to maintain a better electric contact condition in the electrically conductive adhesive 21A by providing a zener diode for minimum required addition. Further, a period of time for lighting the fluorescent bulb (the light source 41) may be in the range of a few seconds to several dozen seconds. Anyway, because the fluorescent bulb (the light source 41) is not full-time turned on, rise in temperature in the storage member 50 may not be introduced, or if a very small rise in temperature is introduced by any possibility, performance of the detector 21 is absolutely unaffected.

The electric contact in the electrically conductive adhesive 21A has a significant effect on characteristics of the detector 21, i.e. resolution of energy and accuracy of time and causes deterioration, therefore maintenance of a better condition of it may contribute largely to the securement of the characteristics of the detector 21. In this embodiment, the electric contact can be maintained in a better condition by the current source unit 40, and therefore even if the detector 21 is formed by lamination of the detector element 211 and the conductive member 22, 23, it may be less deteriorated. Also, the achieved securement of the better electric contact may allow the detector element 211 to be made thinner to be formed and laminated, which may improve both of performance and sensitivity of the detector 21.

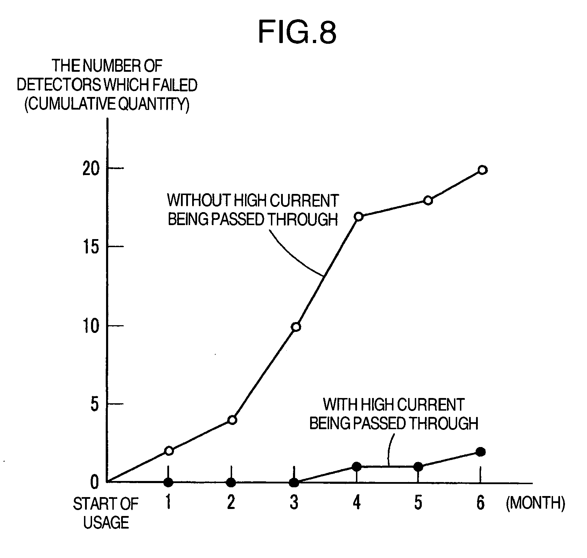

Now, since a high current is passed through every day at startup of the PET imaging device 1 by the current source unit 40, then occurrence of failure (for example, failure to detect gamma radiation or generation of a noise) in the detector 21 can be considerably reduced. Fig. 8 shows a graph for illustrating relation between occurrence of failure in the detector 21 and the number of days (a figure denotes a month), and for half a year (over six months), the high current is passed through every day at startup of the PET imaging device 1, then it is confirmed that there are a total of two occurrences of failure for half a year. Therefore, compared to the case where the high current is not passed through in the PET imaging device 1 (the case without the high current being passed through), in this case, 20 failures in the detector 21 occur for half a year, then it can be understood that the high current being passed through by the current source unit 40 in this embodiment brings considerably successful results for maintaining performance of the detector 21. Accordingly, reliability of the PET imaging device 1 can be greatly improved.

Advantages provided by this embodiment will be explained hereinafter.
(1) In this embodiment, because the current source unit 40 which may force a larger current than that due to a charge generated by incoming gamma radiation (radiation) to flow at least through the electrically conductive adhesive 21A is provided, the large amount of current passed by the current source unit 40 may allow electrically conductive particles in the electrically conductive adhesive 21A to recombine with one another or an oxidized film formed in the electrically conductive adhesive 21A to be destroyed, to stabilize electric contact properties of the electrically conductive adhesive 21A.
(2) Because the protection circuit 31 is provided, the detection circuit 30 can be protected from the current passed by the current source unit 40 to stabilize electric contact properties and also improve reliability of the electrically conductive adhesive 21A.
(3) It is not necessary to use a capacitor 26 having a higher voltage rating, a capacitor 26 having a low voltage rating may be used as-is, and minimum required addition of the protection circuit 31 can protect the existing detection circuit 30 to provide a nuclear medicine imaging system excellent in resolution of energy.
(4) The high current being passed through by the current source unit 40 before application of a voltage for measurement may allow occurrence of failure in the detector 21 to be effectively suppressed, which can maintain detection performance of radiation over a long period of time.
(5) The protection circuit 31 includes any one of a zener diode, selector switch and varistor intervening between a signal output side (anode A side) of the detector element 211 of the detector 21, or any combination thereof, and therefore it is easily possible at a low cost to provide the protection circuit 31. Especially, in the PET imaging device 1 which uses several tens of thousands of the detector elements 211 to hundreds of thousands, accordingly, addition of an expensive component may drive up costs to a large extent, but a nuclear medicine imaging system of this embodiment which will not lead to such a state is useful.
   Moreover, it is not necessary to alter widely the detection circuit 30, a good resolution of energy is attainable, and alteration of wiring structure and the like in the unit board U is required with a minimum.
(6) The electric contact properties of the electrically conductive adhesive 21A may be maintained in a better condition, therefore the detector element 211 may be made thinner to be formed and laminated, which can improve both of performance and sensitivity of the detector 21. In the PET imaging device 1, it is necessary to capture effectively gamma radiation of 511 keV, and for that purpose, a thicker thickness of the detector element 211 is required. However, the thicker thickness of the detector element 211 may lead to a longer traveling distance of an electron and hole, therefore resolution of energy and measuring accuracy of incoming timing may deteriorate. Lamination of many detector elements 211 having a thinner thickness may allow a traveling distance of an electron and hole to be shortened, therefore resolution of energy and measuring accuracy of irradiation timing may improve, and an advantage may be also provided that a large volume occupation ratio of the detector element 211 may be obtained and a volume of the detector 21 may be increased, thereby improving performance of the PET imaging device 1.
(7) In the detector 21, the cathodes C of adjacent detector elements 211 are disposed to face one another and the anodes A are disposed to face one another, therefore the conductive members 22 and 23 may be shared. Further, the electric contact properties of the electrically conductive adhesive 21A are maintained, therefore under a stable condition, the reverse bias voltage may be applied between the electrodes of the cathode C and anode A for collecting charges. Also, it is not necessary to dispose an electrically insulating material between the detector elements 211, and therefore, dense arrangement of the detector elements 211 may be achieved. This can provide improvement in sensitivity and also shortening of the inspection time.

### (Embodiment 2)

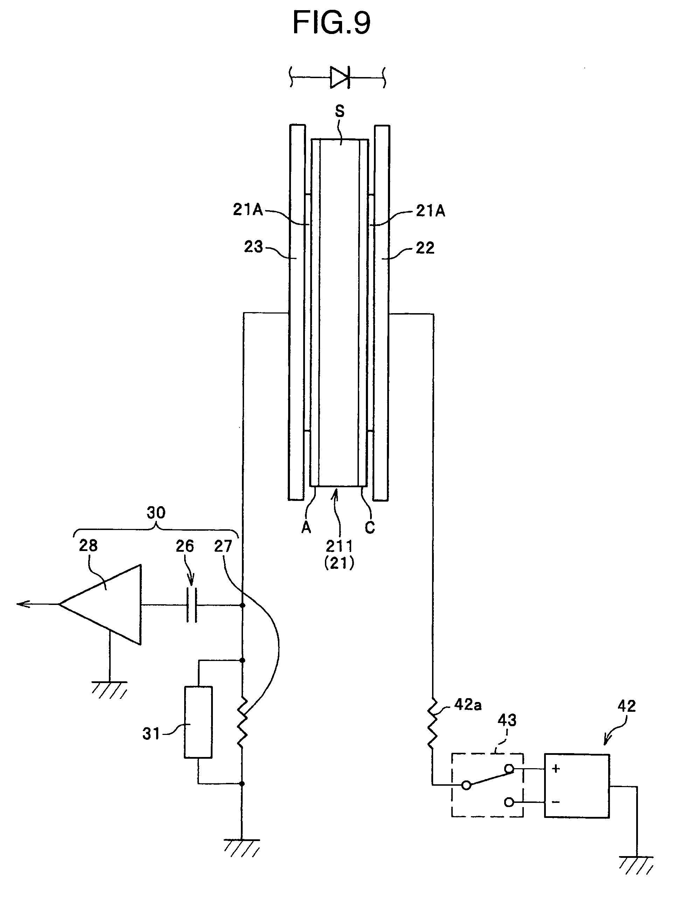

A current source unit applied to a PET imaging device of another embodiment according to the present invention will be explained as an embodiment 2. In this embodiment, as shown in Fig. 9, a selector switch 43 is provided on the output side of the high voltage supply 42 for the current source unit, and the light source 41 described above (see Fig. 6) becomes unnecessary.

The selector switch 43 serves to switch between the reverse direction and forward direction of a bias voltage to the detector 21, and at startup of the PET imaging device 1, it applies a forward bias voltage to the detector 21 to cause a larger current than that generated by incoming gamma radiation on the detector 21 to flow through the electrically conductive adhesive 21A in the detector 21. That is, in this embodiment, a unit for forcing a large current to flow through the detector 21 is only different than that in the embodiment 1 above, and an achievable effect is the same as the embodiment 1 above.

Next, an effect performed by the current source unit before startup of the PET imaging device 1 (see Fig. 1) will be explained with reference to Fig. 9. First, before starting the PET imaging device 1, the forward bias voltage is applied to the detector 21 by actuating the selector switch 43 of the high voltage supply 42 through operation on a control panel (not shown) and the like to connect the minus side of the high voltage supply 42 to the detector 21. In this example, the forward bias voltage is applied for about 10 sec. Further, these operations may be automatically performed according to a preset program (not shown) before a normal startup.

The forward bias voltage applied allows a larger current than that generated by incoming gamma radiation to flow in the detector element 211 of the detector 21. For that matter, also in this embodiment, approximately similarly to the embodiment 1 above, current of about 10 microamperes can be passed through the electrically conductive adhesive 21A in the detector 21.

Such a large amount of current flowing through the detector 21 may allow electrically conductive particles contained in the electrically conductive adhesive 21A to recombine with one another or an oxidized film formed in the electrically conductive adhesive 21A to be destroyed, to stabilize the electric contact properties.

Further, an effect of the zener diode of the protection circuit 31 may lower the voltage across the resistor 27, and therefore, the voltage across the capacitor 26 may be kept below the voltage rating, thereby protecting the detection circuit 30 similarly to the embodiment 1 above.

In this embodiment, the advantages (1) to (7) provided by the embodiment 1 above may be obtained, and further an advantage described below may be achieved.
(8) In this embodiment, minimum necessary and inexpensive additional provision of the selector switch 43 for applying the forward bias voltage from the high voltage supply 42 to the detector 21 and the protection circuit 31 provide a nuclear medicine imaging system excellent in resolution of energy.

Although the embodiments above have been explained in relation to the PET imaging device 1 (see Fig. 1) as an example of a nuclear medicine imaging system, the detector 21 and the detection module 20A of these embodiments of the present invention, not to be limited to the PET imaging device 1, may be applicable to a single photon emission computed tomographic equipment (SPECT equipment) and gamma camera. For that matter, the PET imaging device and SPECT equipment have a common performance of taking up functional image of the subject in 3-dimension, but the SPECT equipment employs measurement of a single photon as measurement principle, therefore it can not measure coincidently and is provided with a collimator for controlling an incident position (angle) of gamma radiation. Also, for the gamma camera, attainable functional image is in 2-dimension and a collimator for controlling an incident angle of gamma radiation is provided. Further, the detector 21 has been described such that it surrounds the bed B and is not moved, but the detector 21 may be moved around the bed B to detect radiation and the field of view 1a may be open.

Moreover, a nuclear medicine imaging system may be configured by combination of the PET imaging device or SPECT equipment with an X-ray CT.

It should be further understood by those skilled in the art that although the foregoing description has been made on embodiments of the invention, the invention is not limited thereto and various changes and modifications may be made without departing from the spirit of the invention and the scope of the appended claims.

## Claims

1. A nuclear medicine imaging system, being configured so that a semiconductor element (S) and a metallic conductive member (22, 23) are bonded to one another with an electrically conductive adhesive (21A) composed of electric conductive particles and a resin binder, and a charge which is generated when radiation is incident on the semiconductor element (S) is taken as a signal by a detection circuit (30) from the conductive member (22, 23) through the electrically conductive adhesive (21A), wherein
a current source unit (40) for forcing a larger current than that due to a charge generated by incoming radiation to flow at least through the electrically conductive adhesive (21A) is provided; and
a protection circuit (31) for protecting the detection circuit (30) from the current passed by the current source unit (40) is provided.

2. The nuclear medicine imaging system according to claim 1, wherein a high current is passed through by the current source unit (40) before application of a voltage for measurement.

3. The nuclear medicine imaging system according to claim 1, wherein the current source unit (40) irradiates the semiconductor element (S) with a light beam and concurrently applies a voltage to the semiconductor element (S).

4. A nuclear medicine imaging system, being configured so that a semiconductor element (S) having a diode characteristic and a metallic conductive member (22, 23) are bonded to one another with an electrically conductive adhesive (21A) composed of electric conductive particles and a resin binder, and a charge which is generated when radiation is incident on the semiconductor element (S) is taken as a signal by a detection circuit (30) from the conductive member (22, 23) through the electrically conductive adhesive (21A), wherein
a current source unit (40) for forcing a larger forward current than that due to a charge generated by incoming radiation to flow at least through the electrically conductive adhesive (21A) is provided; and
a protection circuit (31) for protecting the detection circuit (30) from the forward current passed by the current source unit (40) is provided.

5. The nuclear medicine imaging system according to claim 4, wherein the current source unit (40) applies a forward bias voltage to the semiconductor element (S).

6. The nuclear medicine imaging system according to claim 1, wherein the protection circuit (31) comprises any one of a zener diode, selector switch and varistor intervening between a signal output side of the semiconductor element (S) and the ground side, or any combination thereof.

7. The nuclear medicine imaging system according to claim 1, wherein a radiation detection module (20A) comprises a semiconductor radiation detector (21) formed by laminating alternately the semiconductor element (S) and the conductive member (22, 23).

8. A nuclear medicine imaging method, being configured so that a semiconductor element (S) and a metallic conductive member (22, 23) are bonded to one another with an electrically conductive adhesive (21A) composed of electric conductive particles and a resin binder, and a charge which is generated when radiation is incident on the semiconductor element (S) is taken as a signal by a detection circuit (30) from the conductive member (22, 23) through the electrically conductive adhesive (21A), wherein
a larger current than that due to a charge generated by incoming radiation is forced to flow at least through the electrically conductive adhesive (21A); and
the detection circuit (30) is protected from the larger current than that due to a charge generated by incoming radiation by a protection circuit (31).

9. The nuclear medicine imaging method according to claim 8, wherein when the larger current than that due to a charge generated by incoming radiation is passed at least through the electrically conductive adhesive (21A), it is carried out before application of a voltage for measurement.

10. The nuclear medicine imaging method according to claim 8, wherein the protection circuit (31) comprises any one of a zener diode, selector switch and varistor intervening between a signal output side of the semiconductor element (S) and the ground side, or any combination thereof.
